# EUROPEAN PATENT SPECIFICATION

(11) **EP 2 988 602 B1**
(45) Date of publication and mention of the grant of the patent: **12.12.2018**
(21) Application number: 14719305.6
(22) Date of filing: 23.04.2014
(51) Int. Cl.: A23C 9/123, C12N 15/74, C12P 39/00

(54) **LACTIC ACID BACTERIA**
MILCHSÄUREBAKTERIEN
BACTÉRIES D'ACIDE LACTIQUE

(30) Priority: 23.04.2013 EP 13164936
(43) Date of publication of application: 02.03.2016
(73) Proprietor: DSM IP Assets B.V., 6411 TE Heerlen (NL)
(72) Inventor: MALJAARS, Cornelia Elizabeth Paulina, 6100 AA Echt (NL); DE-GUEMBECKER, Daphne, 6100 AA Echt (NL); PRICE, Claire Emile, 6100AA Echt (NL); VER LOREN VAN THEMAAT, Pieter Emiel, 6100AA Echt (NL); GRABINSKI, Dominik Bohdan, 6100AA Echt (NL)
(74) Representative: DSM Intellectual Property
(86) International application number: PCT/EP2014/058236
(87) International publication number: WO 2014/173947

(56) References cited:
- EP-A1- 0 575 010
- EP-A1- 2 486 124
- WO-A1-2010/103126
- CN-B- 102 090 449
- DEMIRKAYA ALPER KUERSAT ET AL: "The effect of microbial transglutaminase on microbiological, chemical, textural and sensory properties of yogurt", AUSTRALIAN JOURNAL OF DAIRY TECHNOLOGY, DAIRY INDUSTRY ASSOCIATION OF AUSTRALIA, MELBOURNE, AU, vol. 64, no. 2, 1 October 2009 (2009-10-01), pages 171-176, XP009170407, ISSN: 0004-9433

## Description

### Field of the invention

The present invention relates to a composition comprising lactic acid bacteria and a process for manufacturing fermented dairy products using said composition.

### Background of the invention

The food industry uses different bacteria, in the form in particular of ferments, in particular lactic acid bacteria, in order to improve the taste and the texture of foods but also to extend the shelf life of these foods. In the case of the dairy industry, lactic acid bacteria are used intensively in order to bring about the acidification of milk (by fermentation) but also in order to texturize the product into which they are incorporated. Among the lactic acid bacteria used in the food industry, there can be mentioned the genera *Streptococcus* and *Lactobacillus.* The lactic acid bacterial species *Streptococcus thermophilus* and *Lactobacillus delbrueckii* ssp *bulgaricus* are used in particular in the formulation of the ferments used for the production of fermented milks, for example yogurts.

The acidity produced in yogurt depends mainly on the acidifying activity of the yogurt culture (*Streptococcus thermophilus* and *Lactobacillus delbrueckii* ssp. *bulgaricus*) and therefore the amount of lactic acid produced during the milk fermentation and also the residual acidity produced during cold storage. The texture is also varying during storage and participates in the final product sensorial properties. The recipe of the yogurt has also an impact on the yogurt sensorial properties by modifying the texture or the aroma perception.

Yogurt that is available on the consumer market worldwide contains varying amounts of fat. A regular yogurt made from plain (full) or whole (cow) milk may contain between 3-6 wt% fat. A low fat yogurt from half full (cow) milk may contain about half the amount of fat e.g. between 1.5 and 2 wt% (half full yogurt or low fat yogurt). Non-fat yogurt made from skimmed milk (i.e. all fat removed) contains no or very low amounts of fat (0.0 - 0.2 wt%).

Turkish style and Greek style yogurts (also referred to as "strained yogurts") may have much higher fat contents and occasionally also higher protein contents. These yogurts may be produced by removing an excess part of the whey by straining.

Like many types of yogurt, strained yogurt is often made from milk that has been enriched by boiling off some of its water content and/or by adding extra butterfat and powdered milk. Due to the straining process to remove excess whey, even low fat or non-fat varieties of strained yogurt are much thicker, richer, and creamier than the conventional/unstrained yogurts which results in a rapidly expanding popularity of strained yogurts.

WO2010103126 relates to a method for producing an acidified milk product with improved texture and pleasant mouthfeel, wherein an exopolysaccharide producing lactic acid bacteria is used in combination with a transglutaminase enzyme.

There is a consumer need to avail of food products with less fat. Therefore, there is a need for a yogurt which has a much lower fat content than the yogurts now available on the market, for instance there is a need for a Turkish style or a Greek style yogurt with less than 5-10 wt% or a regular yogurt with less than the 3-4 wt% fat, preferably as low as possible, while having the thickness, richness and creaminess of the corresponding yogurt with the high fat content. But simply removing (part of the) fat in a yogurt modifies its properties and for instance maintaining the yogurt texture, as may be expressed by thickness and creaminess, may be a problem. Various food additives have been identified to restore the rheological properties and mouthfeel of these reduced-fat products to those of their full-fat counterparts.

The inventors have now surprisingly found that new selected lactic acid bacteria are capable of improving the one or more of the texture attributes of fermented milk products such as yogurt in comparison to lactic acid bacteria that are known in the prior art. In addition the selected lactic acid bacteria are also capable of partially or fully restoring the texture of a low fat fermented milk products such as yogurt to the texture of higher or full fat fermented milk products such as yogurt.

### Definitions

The term **"milk"** is intended to encompass milks from mammals and plant sources or mixtures thereof. Preferably, the milk is from a mammal source. Mammal sources of milk include, but are not limited to cow, sheep, goat, buffalo, camel, llama, mare and deer. In an embodiment, the milk is from a mammal selected from the group consisting of cow, sheep, goat, buffalo, camel, llama, mare and deer, and combinations thereof. Plant sources of milk include, but are not limited to, milk extracted from soy bean, pea, peanut, barley, rice, oat, quinoa, almond, cashew, coconut, hazelnut, hemp, sesame seed and sunflower seed. In addition, the term "milk" refers to not only whole milk, but also skim milk or any liquid component derived thereof.

As used in the present specification, the term **"fermented milk product"** refers to a product that has been fermented with lactic acid bacteria such as *Streptococcus thermophilus* and optionally *Lactobacillus delbruekii* subsp. *bulgaricus,* but also, optionally, other microorganisms such as *Lactobacillus delbruekii* subsp. *lactis, Bifidobacterium animalis* subsp. *lactis, Lactococcus lactis, Lactobacillus acidophilus* and *Lactobacillus casei,* or any microorganism derived therefrom. The lactic acid strains other than *Streptococcus thermophilus* and *Lactobacillus delbruekii* subsp. *bulgaricus,* are intended to give the finished product various properties, such as the property of promoting the equilibrium of the flora. The fermentation process increases the shelf-life of the product while enhancing and improving the digestibility of milk. Many different types of fermented milk products can be found in the world today. Examples are soured milk (e.g. buttermilk), soured cream and yogurt.

As used herein, the term **"yogurt"** is a fermented milk product produced by fermentation of milk by lactic acid bacteria, also known as "yogurt cultures". The fermentation of the lactose in the milk produces lactic acid which acts on the milk protein to give the yogurt its texture. Yogurt may be made from cow milk, the protein of which mainly comprises casein, which is most commonly used to make yogurt, but milk from sheep, goat, buffalo, camel, llama, mare, deer, water buffalo, ewes and/or mares, and combinations thereof may be used as well. The term "yogurt" furthermore encompasses, but is not limited to, yogurt as defined according to French and European regulations, e.g. coagulated dairy products obtained by lactic acid fermentation by means of specific thermophilic lactic acid bacteria only (i.e. *Lactobacillus delbruekii* subsp. *bulgaricus* and *Streptococcus thermophilus*) which are cultured simultaneously and are found to be living in the final product in an amount of at least 10 million CFU (colony-forming unit) per gram of the yogurt. Preferably, the yogurt is not heat-treated after fermentation. Yogurts may optionally contain added dairy raw materials (e.g. cream and/or protein) or other ingredients such as sugar or sweetening agents, one or more flavouring(s), cereals or nutritional substances, especially vitamins, minerals and fibers. Such yogurt advantageously meets the specifications for fermented milks and yogurts of the AFNOR NF 04-600 standard and/or the codex StanA-Ila-1975 standard. In order to satisfy the AFNOR NF 04-600 standard, the product must not have been heated after fermentation and the dairy raw materials must represent a minimum of 70 wt% of the finished product.
Yogurt encompasses set yogurt, stirred yogurt, drinking yogurt, Petit Suisse, heat treated yogurt and yogurt-like products. Preferably, the yogurt is a stirred yogurt or a drinking yogurt. More preferably, the yogurt is a stirred yogurt.

The term **"starter culture composition"** or **"composition"** (also referred to as **"starter"** or **"starter culture"**) as used herein refers to a composition comprising one or more lactic acid bacteria, which are responsible for the acidification of the milk base. Starter cultures compositions may be fresh (liquid), frozen or freeze-dried. Freeze dried cultures need to be regenerated before use. For the production of a fermented dairy product, the starter cultures composition is usually added in an amount from 0.01 to 3%, preferably from 0.01 and 0.02 % by weight of the total amount of milk base.

As used herein, the term **"lactic acid bacteria"** (LAB) or **"lactic bacteria"** refers to food-grade bacteria producing lactic acid as the major metabolic end-product of carbohydrate fermentation. These bacteria are related by their common metabolic and physiological characteristics and are usually Gram positive, low-GC, acid tolerant, non-sporulating, non-respiring, rod-shaped bacilli or cocci. During the fermentation stage, the consumption of lactose by these bacteria causes the formation of lactic acid, reduces the pH and leads to the formation of a (milk) protein coagulum. These bacteria are thus responsible for the acidification of milk and for the texture of the fermented milk product. As used herein, the term "lactic acid bacteria" or "lactic bacteria" encompasses, but is not limited to, bacteria belonging to the genus of *Lactobacillus* spp., *Bifidobacterium* spp., *Streptococcus* spp., *Lactococcus* spp., such as *Lactobacillus delbruekii* subsp. *bulgaricus, Streptococcus thermophilus, Lactobacillus lactis, Bifidobacterium animalis, Lactococcus lactis, Lactobacillus casei, Lactobacillus plantarum, Lactobacillus helveticus, Lactobacillus acidophilus* and *Bifidobacterium breve.*

The term **"improvement"** or **"improved"** as used in improvement of one or more of the attributes related to texture as defined herein below, means an improvement of one or more of the attributes related to texture obtained while using the composition of the invention as defined herein below in comparison with a composition comprising lactic acid bacteria other than at least strain B or at least strain D, or at least the combination of strain B and strain D. In the Examples such a composition has been used as the Reference. A control experiment without lactic acid bacteria is of course meaningless since in that case no fermented milk product such as yogurt can be obtained and no comparison can be made. An improvement in one or more of the attributes related to texture may be measured absolutely for instance in the case of Brookfield (Pa*s units) or shear stress (Pa units) or more relatively by a taste panel for instance for all the sensory aspects of the fermented milk product.

### Detailed description of the invention

In a first aspect the invention provides a composition comprising *Streptococcus thermophilus* DS71586 (strain B) and *Streptococcus thermophilus* DS71585 (strain D) as defined in the MATERIALS and METHODS. One preferred embodiment of the invention is a composition comprising at least strain B. Another preferred embodiment of the invention is a composition comprising at least strain D. A highly preferred embodiment of the invention is a composition comprising at least strain B and strain D.

The advantage of the compositions of the invention is that strain B as well as strain D is capable of improving the texture of a fermented milk product such as yogurt. In particular, strain B as well as strain D is capable of improving one or more attributes that are related to texture such as the mouthfeel (e.g. the creaminess and/or the thickness), the (visual) structure (e.g. shininess, ropiness and/or visual thickness) and/or the shear stress (e.g. the Brookfield viscosity). It has furthermore surprisingly been found that in compositions comprising both strains B and strain D these improvements are synergistic. Synergistic means that the total effect of the combination of strain B + strain D is more than the sum of the effects of strain B and strain D strain alone. The compositions of the invention are not only capable of improving the texture of the fermented milk products such as yogurt as *such*, but in particular the compositions of the invention are capable of improving and/or partially or fully restoring the texture of the fermented milk product such as yogurt wherein the fat content has been reduced, to the texture of the fermented milk product such as yogurt wherein the fat content not has been reduced, e.g. a high(er) fat or full fat yogurt.

The compositions of the invention may further comprise other lactic acid bacterial strains as defined hereinbefore such as one or more lactic acid bacterial strains selected from the group consisting of *Lactobacillus* spp., *Bifidobacterium* spp., *Streptococcus* spp., *Lactococcus* spp., such as *Lactobacillus delbruekii* subsp. *bulgaricus, Streptococcus thermophilus or Streptococcus thermophilus, Lactobacillus lactis, Bifidobacterium animalis, Lactococcus lactis, Lactobacillus casei, Lactobacillus plantarum, Lactobacillus helveticus, Lactobacillus acidophilus* and *Bifidobacterium breve.*

Preferably the compositions of the invention further comprise one or more other *Streptococcus thermophilus* strains or one or more other *Lactobacillus delbrueckii* ssp. *bulgaricus* strains. These strains may be added because they may have other properties that are advantageous in for instance a process for the production of a fermented milk product such as yogurt or in the final properties of the fermented milk product such as yogurt. These strains may for instance further improve the acidification speed or they may confer certain flavours.

In a preferred embodiment, the compositions of the invention may further comprise *Streptococcus thermophilus* strain A and/or *Streptococcus thermophilus* strain C and preferably *Streptococcus thermophilus* strain A and *Streptococcus thermophilus* strain C. The composition of the invention may further comprise a *Lactobacillus delbrueckii* ssp. *bulgaricus* strain, preferably *Lactobacillus delbrueckii* ssp. *bulgaricus* strain E.

A highly preferred embodiment of the composition of the invention comprises *Streptococcus thermophilus* strain A and *Streptococcus thermophilus* strain B and *Streptococcus thermophilus* strain C and *Streptococcus thermophilus* strain D and *Lactobacillus delbrueckii* ssp. *bulgaricus* strain E.

Preferred compositions of the invention are the following:
5. Composition comprising at least *Streptococcus thermophilus* strain B and strain D
6. Composition comprising at least *Streptococcus thermophilus* strain B and strain D and a *Lactobacillus delbrueckii* ssp. *bulgaricus* preferably strain E.
19. Composition comprising at least *Streptococcus thermophilus* strain B and strain D and strain A.
20. Composition comprising at least *Streptococcus thermophilus* strain B and strain D and strain A and a *Lactobacillus delbrueckii* ssp. *bulgaricus* preferably strain E.
21. Composition comprising at least *Streptococcus thermophilus* strain B and strain D and strain C.
22. Composition comprising at least *Streptococcus thermophilus* strain B and strain D and strain C and a *Lactobacillus delbrueckii* ssp. *bulgaricus* preferably strain E.
23. Composition comprising at least *Streptococcus thermophilus* strain A and strain B and strain C and strain D.
24. Composition comprising at least *Streptococcus thermophilus* strain A and strain B and strain C and strain D and a *Lactobacillus delbrueckii* ssp. *bulgaricus* preferably strain E.
25. A highly preferred composition is composition 25 as defined in Table 2.
26. A highly preferred composition is composition 26 as defined in Table 2.
27. A highly preferred composition is composition 27 as defined in Table 2.
28. A highly preferred composition is composition 28 as defined in Table 2.
29. A highly preferred composition is composition 29 as defined in Table 2.
30. A highly preferred composition is composition 30 as defined in Table 2.

The compositions of the invention may further encompass different embodiments depending on the amount of the strains present in the composition. The amount of the bacterial strain present in the composition may be expressed as cfu's (colony forming units). These cfu's may be expressed relative to the weight (dry or wet weight) of the composition or the volume of the composition. The total amount of cfu's of the various bacterial strains present in the composition of the invention may be set to 100% and the amount of each individual strain present in the composition may be expressed as a % of the total cfu's.

In the compositions of the invention which comprise only strain B or D, these strains constitute 100% of the cfu's. These compositions may be referred to as pure cultures. In the composition comprising both strain B and strain D and no other strains, the strains may be present in any ratio and/or percentage of the total cfu's. The cfu's for strain B:strain D may be (in % of the total cfu's) 1:99 or 2:98 or 3:97 or 4:96 or 5:95 or 6:94 or 7:93 or 8:92 or 9:91 or 10:90 or 11:89 or 12:88 or 13:87 or 14:86 or 15:85 or 16:84 or 17:83 or 18:82 or 19:81 or 20:80 or 21:79 or 22:78 or 23:77 or 24:76 or 25:75 or 26:74 or 27:73 or 28:72 or 29:71 or 30:70 or 31:69 or 32:68 or 33:67 or 34:66 or 35:65 or 36:64 or 37:63 or 38:62 or 39:61 or 40:60 or 41:59 or 42:58 or 43:57 or 44:56 or 45:55 or 46:54 or 47:53 or 48:52 or 49:51 or 50:50 or 51:49 or 52:48 or 53:47 or 54:46 or 55:45 or 56:44 or 57:43 or 58:42 or 59:41 or 60:40 or 61:39 or 62:38 or 63:37 or 64:36 or 65:35 or 66:34 or 67:33 or 68:32 or 69:31 or 70:30 or 71:29 or 72:28 or 73:27 or 74:26 or 75:25 or 76:24 or 77:23 or 78:22 or 79:21 or 80:20 or 81:19 or 82:18 or 83:17 or 84:16 or 85:15 or 86:14 or 87:13 or 88:12 or 89:11 or 90:10 or 91:9 or 92:8 or 93:7 or 94:6 or 95:5 or 96:4 or 97:3 or 98:2 or 99:1 and preferably is 50:50. The skilled person is very well capable, without undue burden, to determine the amounts of strains B and D in a composition of the invention in order to achieve a desired improvement in the properties of the fermented milk product such as yogurt or in the process to produce such a fermented milk product. In the compositions that are comprising other bacterial strains in addition strains B, D or B and D to the total cfu's relate not only to the strains B, D or B+D present in the composition but also to the other bacterial strains present in the compositions of the invention.

*Lactobacillus delbrueckii* ssp. *bulgaricus* strain is a classical yogurt strain and may be present in the composition of the invention. The inventors have found, however, that the *Lactobacillus delbrueckii* ssp. *bulgaricus* strain did not contribute to (the improvements of) any of the texture attributes. Yogurts made with a composition of the invention that is lacking a *Lactobacillus delbrueckii* ssp. *bulgaricus* gave the same values and/or scores of the texture attributes compared to the same composition comprising a *Lactobacillus delbrueckii* ssp. *bulgaricus,* such as strain E.

*Lactobacillus delbrueckii* ssp. *bulgaricus,* when present in the compositions of the invention, preferably strain E (*Lactobacillus delbrueckii* ssp. *bulgaricus* DS71836) may constitute between 0.1% and 10% of the total cfu's of the composition, preferably between 0,2% and 5%, more preferably between 0,5% and 2%, more preferably between 0,8 and 1,2%, most preferably 1%. In the compositions of the invention comprising further *Streptococcus thermophilus* strains, preferably strain B or D or A+B or B+C or A+D or C+D or B+D or A+B+C or A+C+D or A+B+D or B+C+D or A+B+C+D may constitute the remaining cfu's of the composition of the invention. The skilled person is very well capable, without undue burden, to determine the amounts of strains A and/or B and/or C and/or D and/or E in the composition of the invention in order to achieve a desired improvement in the texture of the fermented milk product such as yogurt or in the process to produce such a fermented milk product. In the compositions that are comprising other bacterial strains the total cfu's relates not only to the strains strains A and/or B and/or C and/or D and/or E present in the various compositions but also to the other bacterial strains present in the compositions of the invention.

In the composition comprising both strain B and strain D and strain E, the strains may be present in any ratio and/or percentage of the total cfu's. In a preferred embodiment strains E is present as 1% based on cfu's. This means that the sum of strains B and D amount to 99%. The cfu's for strain B:strain D may then be (in % of the total cfu's) 1:98 or 2:97 or 3:96 or 4:95 or 5:94 or 6:93 or 7:92 or 8:91 or 9:90 or 10:89 or 11:88 or 12:87 or 13:86 or 14:85 or 15:84 or 16:83 or 17:82 or 18:81 or 19:80 or 20:79 or 21:78 or 22:77 or 23:76 or 24:75 or 25:74 or 26:73 or 27:72 or 28:71 or 29:70 or 30:69 or 31:68 or 32:67 or 33:66 or 34:65 or 35:64 or 36:63 or 37:62 or 38:61 or 39:60 or 40:59 or 41:58 or 42:57 or 43:56 or 44:55 or 45:54 or 46:53 or 47:52 or 48:51 or 49:50 or 50:49 or 51:48 or 52:47 or 53:46 or 54:45 or 55:44 or 56:43 or 57:42 or 58:41 or 59:40 or 60:39 or 61:38 or 62:37 or 63:36 or 64:35 or 65:34 or 66:33 or 67:32 or 68:31 or 69:30 or 70:29 or 71:28 or 72:27 or 73:26 or 74:25 or 75:24 or 76:23 or 77:22 or 78:21 or 79:20 or 80:19 or 81:18 or 82:17 or 83:16 or 84:15 or 85:14 or 86:13 or 87:12 or 88:11 or 89:10 or 90:9 or 91:8 or 92:7 or 93:6 or 94:5 or 95:4 or 96:3 or 97:2 or 98:1 and preferably is 49.5:49.5 and 1% strain E (i.e. Composition 30).

The skilled person is very well capable, without undue burden, to determine the amounts of strains B and D in a composition of the invention in order to achieve a desired improvement in the properties of the fermented milk product such as yogurt or in the process to produce such a fermented milk product.

In a second aspect, the invention provides the following bacterial strains of the invention:
Strain A. *Streptococcus thermophilus* DS71579 deposited with the Centraalbureau voor Schimmelcultures on 09 April 2013 having deposition number CBS134831.
Strain B. *Streptococcus thermophilus* DS71586 deposited with the Centraalbureau voor Schimmelcultures on 09 April 2013 having deposition number CBS134834.
Strain C. *Streptococcus thermophilus* DS71584 deposited with the Centraalbureau voor Schimmelcultures on 09 April 2013 having deposition number CBS134832.
Strain D. *Streptococcus thermophilus* DS71585 deposited with the Centraalbureau voor Schimmelcultures on 09 April 2013 having deposition number CBS134833.
Strain E. *Lactobacillus delbrueckii* ssp. *bulgaricus* DS71836 deposited with the Centraalbureau voor Schimmelcultures on 9 April 2013 having deposition number CBS134835.

In a third aspect the invention provides a process for the production of a fermented milk product, preferably yogurt, comprising fermenting milk using any of the compositions as defined by the first aspect of the invention and wherein the texture of the fermented milk product obtained has been improved compared to the texture of a fermented milk product that has not been produced using any of the compositions as defined by the first aspect of the invention or in the Materials and Methods.

A preferred composition of the invention that may be used in the process of the invention is a composition comprising at least strain B and/or strain D selected from the group consisting of 5 and composition 6 and composition 19 and composition 20 and composition 21 and composition 22 and composition 23 and composition 24 and composition 25 and composition 26 and. Preferred compositions are compositions comprising at least strain B and D such as composition 5 or composition 6 or composition 19 or composition 20 or composition 21 or composition 22 or composition 23 or composition 24 or composition 25 or composition 26 or composition 27 or composition 30. Most preferred is composition 30. The advantage of the process of the invention comprising using compositions comprising at least strain B and D is that strain B and D have a synergistic effect in the improvement of the texture of the fermented milk product, preferably yogurt, and in particular a synergistic effect in the improvement of the rheology attributes shear stress and Brookfield viscosity.

In one embodiment, the invention provides a process of the invention wherein one or more textural attributes selected from the group consisting of rheology, appearance, the structure, mouthfeel, the after feel of the fermented milk product, preferably yogurt, has been improved. Most preferably, the invention provides a process wherein the rheology attributes of the fermented milk product, preferably yogurt, more preferably the Brookfield and/or the shear stress has been improved. In another preferred embodiment, the invention provides a process wherein the appearance of the fermented milk product, preferably yogurt, more preferably the shininess and/or the whiteness has been improved. In yet another preferred embodiment, the invention provides a process wherein the structure of the fermented milk product, preferably yogurt, more preferably the visual aspects such as ropiness and/or visual thickness and/or smoothness has been improved. In another preferred embodiment, the invention provides a process wherein the mouthfeel of the fermented milk product, preferably yogurt, more preferably the thickness and/or the creaminess and/or the sliminess and/or the melting and/or the astringency has been improved. In another preferred embodiment, the invention provides a process wherein the after feel of the fermented milk product, preferably yogurt, more preferably the astringency and/or the pungency and/or the fat coating has been improved.

Highly preferably, the invention provides a process of the invention wherein two or more, preferably three or more, more preferably four or more textural attributes selected from the group consisting of Brookfield and the shear stress and the shininess and the whiteness and ropiness and visual thickness and smoothness and the thickness and the creaminess and the astringency and the pungency and the fat coating has been improved.

The most preferred fermented milk product that is produced by the process of the second aspect of the invention is yogurt as defined hereinbefore.

The milk that may be used in the process of the third aspect of the invention, may be any milk suitable for the production of a fermented milk product, such as yogurt. Milk has been defined hereinbefore and may encompass milks from mammals and plant sources or mixtures thereof. Preferably, the milk is from a mammal source. Mammal sources of milk include, but are not limited to cow, sheep, goat, buffalo, camel, llama, mare and deer. In an embodiment, the milk is from a mammal selected from the group consisting of cow, sheep, goat, buffalo, camel, llama, mare and deer, and combinations thereof. Plant sources of milk include, but are not limited to, milk extracted from soy bean, pea, peanut, barley, rice, oat, quinoa, almond, cashew, coconut, hazelnut, hemp, sesame seed and sunflower seed. In addition, the term "milk" refers to not only whole milk, but also skim milk or any liquid component derived thereof. The fat content in the milk and in the subsequent fermented milk product, such as yogurt, may be as is known in the prior and as is referred in the background of the invention.

Preferably, the invention provides a process wherein fermented milk products, such as yogurt, may be produced with fat contents as low as possible, while having texture properties of fermented milk products, such as yogurt, with higher fat contents whereby the process comprises using any of the compositions of the first aspect of the invention which has been described hereinbefore.

In a fourth aspect, the invention provides a fermented milk product, preferably yogurt, obtainable by the process of the third aspect of the invention, comprising a composition as defined in the first aspect of the invention characterized in that the fermented milk product, preferably yogurt, wherein one or more textural properties selected from the group consisting of the appearance, the structure, the mouthfeel, the after feel and the shear stress of the fermented milk product, preferably yogurt, has been improved. Highly preferably, the invention provides a fermented milk product wherein two or more, preferably three or more, more preferably four or more textural properties selected from the group consisting of the appearance, the structure, the mouthfeel, the after feel and the shear stress of the fermented milk product, preferably yogurt, has been improved. Most preferably, the invention provides a fermented milk product wherein the mouthfeel and/or the rheology of the fermented milk product, preferably yogurt, has been improved. Highly preferred improvements are the creaminess and/or the thickness and/or the Brookfield of the fermented milk product.

In a fifth aspect, the invention provides the use of any of the compositions as defined in the first aspect of the invention for the production of the fermented milk product, preferably yogurt, as defined in the fourth aspect of the invention. Most preferred is the use of a composition comprising at least strain B and/or strain D selected from the group consisting of composition 5 and composition 6 and composition 19 and composition 20 and composition 21 and composition 22 and composition 23 and composition 24 and composition 25 and composition 26 and composition 27 and composition 28 and composition 29 and composition 30 whereby more preferred compositions are compositions comprising at least strain B and strain D such as composition 5 or composition 6 or composition 19 or composition 20 or composition 21 or composition 22 or composition 23 or composition 24 or composition 25 or composition 26 or composition 27 or composition 30. Most preferred is composition 30.

### MATERIALS AND METHODS

### 1. Bacterial strains.

Table 1 summarizes the bacterial strains used in the Examples.

**Table 1. Bacterial strains**

| Strain | CBS number | Strain |
|---|---|---|
| A | CBS134831 | *Streptococcus thermophilus* DS71579 |
| B | CBS134834 | *Streptococcus thermophilus* DS71586 |
| C | CBS134832 | *Streptococcus thermophilus* DS71584 |
| D | CBS134833 | *Streptococcus thermophilus* DS71585 |
| E | CBS134835 | *Lactobacillus delbrueckii ssp. bulgaricus* DS71836 |

| | | |
|---|---|---|
| All strains A-E were deposited on 9 April 2013 at the Centraalbureau voor Schimmelcultures (Fungal Biodiversity Centre), Uppsalalaan 8, 3584 CT Utrecht, The Netherlands under the provisions of the Budapest Treaty. | | |

### 2. Compositions comprising bacterial strains

The following compositions were used in the Examples. The percentages relate to the cfu's (colony forming units) - see Table 2.

**Table 2.**

| **Compositions comprising bacterial strains - the % values relate to the cfu's of the respective strain in the composition. The total of the cfu's of the composition is 100%.** | | | | | |
|---|---|---|---|---|---|
| Composition | Strain A | Strain B | Strain C | Strain D | Strain E |
| 25 | 24,75% | 24,75% | 24,75% | 24,75% | 1% |
| 26 | 24,75% | 24,75% | 16,5% | 33,0% | 1% |
| 27 | 25,20% | 25,20% | 16,5% | 33,0% | 0.1% |
| 28 | - | 99,0% | - | - | 1% |
| 29 | - | - | - | 99,0% | 1% |
| 30 | - | 49.5% | - | 49.5% | 1% |

| | | | | | |
|---|---|---|---|---|---|
| The Reference culture (Ref) used in the examples is a commercially available yogurt starter culture and does not contain any of strains A-E. | | | | | |

### 3. Yogurt preparation (all examples)

The fermented milk used is obtained by supplementing pasteurized skimmed milk (Isigny - Example 1 and 2; Campina, The Netherlands - Examples 3-6) with skimmed milk powder and cream (35% fat or 39% fat - see Examples). The final recipe is described below. The milk mixture is pasteurized at 95°C during 6 minutes at 180 bars. The pasteurized milk mix is then inoculated with the culture to be tested at a rate of 0,02% (w/w) and incubated at 38°C in a water bath until a pH of 4,60 is reached. The monitoring of the pH is continuously recorded. Fermented milk then obtained is stirred, smoothened and cooled down to 22°C before filling in the cups. The yogurt cups are then stored at 4°C.

### 4. Yogurt recipes

The following recipes were used in the Examples. All additions are wt% of the total milk recipe.

**Table 3. Yogurt recipes**

| Ingredient | Recipe | | | | | |
|---|---|---|---|---|---|---|
| | A | B | C | D | E | F |
| Skimmed Milk | 76.0 | 71.1 | 78.0 | 73.3 | 96.0 | 87.7 |
| Skimmed Milk Powder | 0.8 | - | 0.4 | - | 0.4 | 1.0 |
| Cream (35% fat) | 15.7 | 21.4 | - | - | - | - |
| Cream (39% fat) | - | - | 14.1 | 19.0 | 3.6 | 3.6 |
| Sucrose | 7.5 | 7.5 | 7.5 | 7.7 | - | 7.7 |
| Fat concentration | 5.5 | 7.5 | 5.5 | 7.5 | 1.4 | 1.4 |

### 5. Texture analysis of yogurt (used in Examples 1 and 2)

During storage, the rheological properties of the samples were measured using a rheometer (Haake VT550 ThermoFischer Scientific) equipped with a Haake vane, Immersion Sensor system FL measuring system and a coaxial cylinder with cup, Sensor System DIN 53019 measuring system. The yogurt viscosity was measured at 20°C with shear rates varying from 0.27 to 300 s⁻¹. Shear rates were increased and then decreased and the upward and downward curves of shear stress (Pascal, Pa) were recorded. For further analysis, shear stress at 300 s⁻¹ was chosen and gave a good correlation to the sensorial evaluation of mouth thickness.

### 6. Sensory analysis of yogurts (used in Examples 1 and 2)

The sensory panel consisted of 8 members who had a specific training in sensory evaluation of yogurts. Products were presented in 3-digit coded, white plastic isothermal cups stored at 4°C. The samples were at approximately 10°C when they were tested. Panellists were provided with mineral water for palate cleaning between samples. The sessions were carried out in a temperature controlled room at 20°C under white lighting in individual booths.

Data acquisition was assisted by FIZZ Sensory Analysis Software. Both monadic and hedonic scales are being used to rate the flavour and the texture attributes of products. The attributes were evaluated in the following order: visual texture with a spoon, texture-in-mouth, taste and aroma.

### 7. Visual aspect of the yogurt (used in Examples 1 and 2)

The aspect of the yogurt is assessed visually by taking a spoonful of yogurt and evaluating if the product is grainy / sandy or smooth.

### 8. Shear stress of yogurt (used in Examples 3-6)

The samples were measured using a Physica MCR501 rheometer equipped with a concentric cylinder measurement system (CC-27). A solvent trap was used to prevent evaporation of water as much as possible. The samples were slightly stirred with a spoon before loading into the rheometer. Before measurement the samples were allowed to rest and heat/cool to the measuring temperature (25°C) for 5 minutes. A standard experimental protocol was applied consisting out of the following two measuring sequences:
1. A strain sweep to determine the initial gel strength (dynamic shear modulus): this is an oscillatory test where at a fixed angular frequency (omega = 10 rad/s) an increasing amplitude is applied: on a logarithmic scale the amplitude is increased from 0.01 to 100 % with 5 measuring points per decade.
2. After the strain sweep the yoghurts are allowed to rest for 30 seconds in the rheometer and subsequently a shear rate sweep is applied to determine the shear stress in mouth: This consists of applying an increasing shear rate to the yoghurts ranging from 0.001 to 1000 s⁻¹ on a logarithmic scale with 3 measuring points per decade (no fixed time setting: the rheometer software determines the required shearing time per measuring point).

This experiment gives a flow curve whereby the measured stress is plotted as a function of the applied shear rate. This curve can then be combined with literature data to determine the relevant shear stress in the mouth as explained in the following.

By sensory panelling of various food products Shama and Sherman identified windows of instrumental shear stresses and shear rates corresponding to products with similar thickness ratings but different shear-thinning behavior. These windows correspond to the rheological regimes applied in the mouth during thickness rating. The governing shear rate was shown to be dependent on the viscosity of the product itself. (see Figure 1 from Shama, F. and Sherman, P. Journal of Texture Studies, 4, 111-118. (1973), "Identification of stimuli controlling the sensory evaluation of viscosity II oral methods*"*).

For the yoghurts of the examples below the (predicted) shear stress in the mouth is determined by plotting the experimentally measured flow curves (measured shear stress in function of applied shear rate of the shear rate sweep experiment described above) onto the aforementioned Figure 1 from Shama and Sherman. The predicted shear stress in the mouth is defined as the cross-over between the measured flow curves and the upper bound of the "shear rate - shear stress" windows of Figure 1 of Shama and Sherman. In figure 2 the authors give examples for various food stuffs. The thus derived shear stress gave a good correlation with the sensory perception of thickness in the mouth.

### 9. Sensory analysis of yogurts (used in Examples 3-6)

Descriptive sensory analysis was done by using the Quantitative Descriptive Analysis Method (Stone, H. and Sidel, J.L. "Sensory Evaluation Practises" 3rd dition, 2004). First, the panellists developed a list of attributes including definitions by means of evaluating a wide variety of references and a wide array of yogurts. Secondly, training sessions were organized to enable panellists to learn to consistently differentiate and replicate the yogurt samples. During the actual QDA measurements the intensities of the selected attributes were obtained per product by the FIZZ (Biosystems; France) sensory data acquisition system, using unstructured line scales ranging from 0 - 100. The products were offered semi-monadically and evaluated twice by the panellists (n=14) by means of a Balanced Complete Block design to avoid sequence effects. Statistical analysis of the data was done by analysis of variance with Fisher's least significant difference (LSD) as a post hoc test (SenPaq) and modelled using Principle Component analysis (PCA) (SenPaq).

### 10. Attributes of stirred yogurt (used in Examples 3-6)

For the **appearance** (i.e. the visual assessment of surface of the yogurt) the following attributes may be determined:
- **Shininess** which is the degree in which the surface of the yogurt reflects the light.
- **Whiteness** which is the whiteness of the surface of the yogurt.

For the **structure** (i.e. the visual assessment of texture by means of spoon) the following attributes may be determined after stirring the yogurt 5 times before assessing the structure.
- **Ropiness** which is the degree in which the yogurt runs from the spoon. The anchors may range from short to long. Short means that there is no formation of threads. Long means that the product forms almost one long thread when it runs from the spoon.
- **Thickness** which is the force needed for stirring the yogurt.
- **Smoothness** which is the degree in which the product is smooth without grittiness, grains and lumps.

For the **odour** the following attributes may be determined
- **Intensity** which is the general and overall odour intensity.
- **Sour** which is the intensity of the sour odour.
- **Sweet** which is the intensity of the sweet odour.
- **Fruity** which is the degree in which a fruity odour is present in the product (e.g. apple, lemon, lime).
- **Off-odour** which is a group of odours which can't be assessed by means of other odour attributes such as manure, stable, cauliflower, sulphur and medicinal odours.

For the **flavour** the following attributes may be determined
- **Intensity** which is the general and overall flavour intensity.
- **Sour** which is the degree in which the product tastes sour (mimicked by citric acid).
- **Milky/creamy** which is the degree in with a milk/cream flavour is perceived (this can be mimicked with full fat milk or cream).
- **Green** which is the degree in which a green flavour is perceived. It can be best described as the green flavour which can be perceived when eating a green apple. This can be mimicked with acetaldehyde.
- **Sweet** which is the degree in which the product has a sweet taste.
- **Bitter** which is the degree in which the product has a bitter taste.

For the **mouthfeel** (assessment of mouthfeel, when the product is inside the mouth) the following attributes may be determined
- **Thickness** which is the degree in which the product feels thick in the mouth. This sensation can be best perceived between tongue and palate.
- **Grainy** which is the degree in which the product feels grainy/powdery in the mouth.
- **Creaminess** which is the full and creamy feeling in the mouth. The product does not feel rough; it does not feel dry and gives a velvety coating in the mouth.
- **Slimy** which is the degree in which the product feels slippery and forms threads in the mouth. This sensation has to be assessed by pushing the product against the palate with the tongue.
- **Melting** which is the degree (slow - quick) in which the product mixes with saliva and disappears out of the mouth.
- **Astringent** which is the degree in which an astringent feeling is present in your mouth after you swallowed the product (the sensation can be mimicked with red wine and the "skin" of a nut).

For the **after taste** (assessment after swallowing the product) the following attributes may be determined
- **Intensity** which is the general and overall aftertaste intensity.
- **UHT** which is the degree in which the aftertaste of sterilized milk (long life milk) is present (e.g. caramel or cooked milk but not burnt milk).
- **Chemical** which is the intensity of a possible chemical aftertaste such as vitamins, rubber and artificial sweeteners.
- **Length** which is the duration (in time) of the aftertaste.

For the **after feel** (assessment after swallowing the product) the following attributes may be determined
- **Astringent** which is the degree in which an astringent feeling is present in the mouth after the product has been swallowed (the sensation can be mimicked with red wine and the "skin" of a nut).
- **Pungent** which is the degree in which a pungent feeling remains after swallowing the yogurt.
- **Fat coating** which is the degree in which a fat coating remains in the mouth after the products is swallowed (filming effect).

### 11. Determination of the pH and the titratable acidity (used in Examples 3-6)

The titratable acidity was determined using an automatic Metrohm titration center consisting of a titrator (736 GP Titrino), a pH electrode (type 6.1110.100) and a 20 ml exchange unit supplied with a 0.1 M sodium hydroxide solution. Approximately 10 g of sample was accurately weighed to within 0.01 g into a 100 ml plastic titration cup and 60 ml of UHQ water (water resistance > 18.2 MOhm/cm, TOC < 500 µg/l) was added with a dispenser. The sample was stirred with a magnetic stirring bar and titrated with 0.1 M sodium hydroxide until pH 8.4 by standard endpoint titration. If the volume of the titrant was below 2 ml or above 20 ml, the analysis was repeated with an adjusted sample amount to obtain a titrant volume of 2-20 ml. The analysis was performed in duplicate. The pH was determined as the pH of the sample prior to starting the titration with sodium hydroxide. The titratable acidity is expressed as % lactic acid or calculated as Dornic degrees (°D). 1°D corresponds to 0.01% (w/v) or 0.1 g/l lactic acid. The number of Dornic degrees is equal to the number of 0.1 ml of a 0.111 M sodium hydroxide solution required to neutralize 10 ml of milk or 10 g of yogurt

### 12. Brookfield (used in Examples 3-6)

Viscosity measurements were performed using a Brookfield RVDVII+ Viscometer, which allows viscosity measurement on an undisturbed product (directly in the pot). The Brookfield Viscometer determines viscosity by measuring the force required to turn the spindle into the product at a given rate. The Helipath system with a T-C spindle was used as it is designed for non-flowing thixotropic material (gels, cream). It slowly lowers or raises a rotating T-bar spindle into the sample so that not always the same region of the sample is sheared (helical path). Thus, the viscometer measures constantly the viscosity in fresh material and is thus considered to be the most suitable for measuring stirred yogurt viscosity. A speed of 30 rpm was used for 31 measuring points, at an interval of 3 seconds. The averages of the values between 60 and 90 seconds are reported.

### 13. Relevance of scores of the texture attributes in all Examples.

The +-signs indicate the average score of the yogurts on each of the attributes ranging from a low (+) to a high (+++) score and include information on significance, e.g. +, ++ and +++ are significantly different from each other, where +++ is significantly different from +/++, but + and ++ are not.

### EXAMPLES

### Example 1

### Effect of lactic acid bacterial strains on various properties of a yogurt.

The milk mixture according to recipe A (Table 3) was preheated to 65°C and homogenized at 180 bars and pasteurized to 95°C for 6 minutes to inactivate any organisms or enzymes present in the milk mix. The milk mixture was then cooled down to about 38°C and inoculated with the compositions as indicated below at 0.02 wt%. The milk mixture containing the culture was then kept at 38°C in a water bath until the pH reached a value of 4.65 - 4.60. Following this step of fermentation, the yogurt was stirred, cooled at 22°C, filled in cups and stored at 4°C to stop the fermentation but not to inactivate or kill the culture.

**Table 4**

| | Composition | | | |
|---|---|---|---|---|
| | Ref | 25 | 26 | 27 |
| Time to reach pH=4.6 (min) | 526 | 318 | 310 | 302 |
| Shear stress at 300 s⁻¹ (Pa)* | 0.254 | 0.251 | 0.260 | 0.262 |
| pH* | 4.46 | 4.12 | 4.11 | 4.13 |
| Acidity* | 81 | 97 | 98 | 95 |
| Mouthfeel* | - | ++ | ++ | ++ |
| Visual aspect* | Smooth | Smooth | Grainy | Grainy |

| | | | | |
|---|---|---|---|---|
| * all measured in the yogurt after 14 days of cold storage of the yogurt | | | | |

The results show that all compositions 25-27 improve the mouthfeel of the yogurt compared to the Reference culture. Only the yogurt obtained with composition 25 has a smooth structure (like the control) while compositions 26 and 27 result in yogurts with a grainy structures.

### Example 2

### Effect of lactic acid bacterial strains on various properties of a yogurt.

Yogurt was made according to recipe A and recipe B as defined in Table 3 according to the method described in Example 1.

**Table 5**

| | Composition | | |
|---|---|---|---|
| | Reference | Reference | 25 |
| Recipe | A | B | A |
| Wt% fat in yogurt | 5.5 | 7.5 | 5.5 |
| Time to reach pH=4,6 (min) | 370 | 342 | 314 |
| Shear stress at 300 s⁻¹ (Pa)* | 0.244 | 0.263 | 0.259 |
| pH* | 4.43 | 4.40 | 4.11 |
| Acidity* | 77 | 72 | 94 |
| Mouthfeel* | + | ++ | ++ |

| | | | |
|---|---|---|---|
| * all measured in the yogurt after 14 days of cold storage of the yogurt | | | |

The results show that in the case of the Reference composition, increasing the fat content in the yogurt from 5.5 wt% (Recipe A) to 7.5% (Recipe B) improves the mouthfeel whereas the other attributes (time to reach pH, viscosity, pH, acidity) remain substantially the same. Furthermore, when using Composition 25 to make a yogurt with a 5.5 wt% fat content, the mouthfeel is improved and is substantially the same as the mouthfeel of the yogurt with 7.5 wt% fat and using the Reference.

### Example 3

### Effect of lactic acid bacterial strains on various properties of a yogurt.

Yogurt was made according to recipe C and recipe D as defined in Table 3 according to the method described in the Materials & Methods (section 3). The two recipes have different fat contents and similar protein content: Recipe C has 5.5% fat and 3.1 wt% protein and Recipe D has 7.5% fat and 2.9 wt% protein.

**Table 6**

| | | Composition | | | |
|---|---|---|---|---|---|
| | | Reference | | Composition 25 | |
| Recipe | | C | D | C | D |
| Wt% fat in yogurt | | 5.5 | 7.5 | 5.5 | 7.5 |
| Time to reach pH=4,6 (min) | | 495 | 450 | 425 | 405 |
| pH* | | 4.71 | 4.74 | 4.49 | 4.48 |
| Rheology* | Shear stress (Pa)* | 27 | 30 | 30 | 32 |
| | Brookfield (Pa*s)* | 14 | 18 | 16 | 19 |
| Appearance* | Shininess* | +/++ | ++ | +/++ | + |
| Structure* | Ropiness* | +++ | ++ | +/++ | + |
| | Thickness* | + | ++ | ++ | +++ |
| Mouthfeel* | Thickness* | + | +/++ | ++ | +++ |
| | Creaminess* | + | ++/+++ | ++ | +++ |
| Flavour* | Sour* | + | + | ++ | ++ |
| | Green* | + | + | ++ | +/++ |

| | | | | | |
|---|---|---|---|---|---|
| * all attributes were measured in the yogurt after 7 days of cold storage of the yogurt. All attributes listed in the Materials and Methods have been measured. Attributes not listed in the table above did not differ significantly for the different yogurts made. | | | | | |

The results show that composition 25, amongst others, improves the rheological properties as well as the mouthfeel attributes thickness comparison with the Reference composition for both the 7.5% and 5.5% fat recipes. For the 5.5% fat recipe, Composition 25 was also assessed to have increased creaminess. Furthermore, when using Composition 25 in a yogurt with a 5.5 wt% fat content, the shear stress, Brookfield as well as the mouthfeel attributes thickness and creaminess are improved and substantially the same as from the yogurt with 7.5 wt% and made using the Reference composition.

### Example 4

### Effect of lactic acid bacterial strains on various properties of a yogurt.

Yogurt was made according to recipe E as defined in Table 3 according to the method described in the Materials & Methods (section 3).

**Table 7**

| | | Composition | | | | |
|---|---|---|---|---|---|---|
| | | Ref | 25 | 28 | 29 | 30 |
| Time to reach pH=4.6 (min) | | 470 | 445 | 445 | 966 | 470 |
| pH* | | 4.5 | 4.23 | 4.31 | 4.45 | 4.39 |
| Rheology* | Shear stress(Pa) | 17 | 21 | 23 | 17 | 27 |
| | Brookfield (Pa*s) | 6.1 | 7.4 | 8.8 | 7.2 | 10.2 |
| Appearance* | Shininess* | ++ | ++ | ++ | + | ++ |
| Structure* | Ropiness* | ++ | ++ | +++ | + | +++ |
| | Thickness* | + | +/++ | +/++ | + | ++/+++ |
| | Smoothness* | ++ | ++ | ++ | + | ++ |
| Mouthfeel* | Thickness* | + | + | ++/+++ | + | ++ |
| | Creaminess* | + | + | ++ | + | ++ |
| | Melting* | ++ | ++ | + | ++ | + |

| | | | | | | |
|---|---|---|---|---|---|---|
| * all measured in the yogurt after 7 days of cold storage of the yogurt. All attributes listed in the Materials and Methods have been measured. Attributes not listed in the table above did not differ significantly for the different yogurts made. | | | | | | |

The results show that composition 28 (comprising strain B+E) improves the rheological attributes shear stress (from 17 to 23 Pa), Brookfield (from 6.1 to 8.8 Pa*s) as well as the mouthfeel attributes creaminess (from + to ++) and creaminess (from + to ++), compared to the Reference composition. The results show that composition 29 (comprising strain D+E) significantly improves the Brookfield (from 6.1 to 7.2 Pa*s) compared to the Reference composition while the other attributes are essentially the same compared to the Reference. The results show that composition 30 (comprising strain B+D+E) improves the rheological attributes shear stress (from 17 to 23 Pa), Brookfield (from 6.1 to 8.8 Pa*s) as well as the mouthfeel attributes thickness (from + to ++) and creaminess (from + to ++), compared to the Reference composition.

Most apparent is the synergistic effect between Strain B and Strain D as observed for Composition 30 when its rheological properties are compared with those of Compositions 28 and 29. For instance, Composition 28 improves the Shear Stress with 6 Pa (from 17 (Reference) to 23 (Composition 28). Composition 29 has the same Shear stress as the Reference. But Composition 30 improves the shear stress with 10 Pa which is more than the sum of 6+0 = 6 Pa (sum of Combination 28 and 29).

The same applies for the Brookfield viscosities: Composition 28 improves from 6.1 to 8.8 Pa*s and Composition 29 improves from 6.1 to 7.2 Pa*s while Composition 30 improves from 6.1 to 10.2 Pa*s (increase of 4.1 Pa*s) which is more than the sum of 2.7+1.1 =3.8 Pa*s.

### Example 5

### Effect of lactic acid bacterial strains on various properties of a yogurt containing sucrose.

Yogurt was made according to recipe F as defined in Table 3 according to the method described in the Materials & Methods (section 3).

**Table 8**

| | | Composition | | | | |
|---|---|---|---|---|---|---|
| | | Ref | 25 | 28 | 29 | 30 |
| Fermentation time | | 495 | 468 | 343 | 1094 | 434 |
| Rheology* | Shear stress (Pa) | 20 | 23 | 24 | 18 | 39 |
| | Brookfield (Pa*s) | 6.5 | 8.6 | 7.8 | 8.1 | 13.8 |

| | | | | | | |
|---|---|---|---|---|---|---|
| * all measured in the yogurt after 7 days of cold storage of the yogurt. | | | | | | |

The results show that composition 28 (comprising strain B+E) improves the rheological attributes shear stress (from 20 to 24 Pa) and the Brookfield (from 6.5 to 7.8 Pa*s) compared to the Reference composition. The results show that composition 29 (comprising strain D+E) improves the Brookfield (from 6.5 to 8.1 Pa*s) compared to the Reference composition. The results show that composition 30 (comprising strain B+D+E) improves the rheological attributes shear stress (from 20 to 39 Pa) and the Brookfield (from 6.5 to 13.8 Pa*s)

Most apparent is the synergistic effect as observed with Composition 30 when its rheological properties are compared with those of Compositions 28 and 29. For instance, Composition 28 improves the shear stress with 4 Pa, composition 29 has a slightly lower shear stress as the Reference. But Composition 30 improves the Shear stress with 19 Pa which is more than the sum of 4-2 = 2 Pa (sum of Combination 28 and 29). The same applies for the Brookfield viscosities: Composition 28 improves with 1.3 Pa*s and Composition 29 improves with 1..6 Pa*s while Composition 30 improves the Brookfield with 7.3 Pa*s which is more than the sum of 1.3+1.6=2.9.

### Example 6

### Synergistic effect of Strain B and Strain D on the texture attributes Brookfield and shear stress.

Yogurt was made according to recipe F as defined in Table 3 according to the method described in the Materials & Methods (section 3).

To study the main effects of strains B and D as well as their interaction on texture, an experiment was designed whereby the Brookfield and shear stress were measured as response variable (see Materials and Methods). These experiments include 2 replicates for each of the four compositions: the Reference, composition 24 (comprising strain B), composition 25 (comprising strain D) and composition 26 (comprising strain B+D). A two-way ANOVA was conducted to find the main and interaction effects on the texture attributes Brookfield and shear stress for strain B and strain D under varying cfu's.

**Table 9**

| Composition | Brookfield Pa*s | Shear stress Pa |
|---|---|---|
| Reference | 6.0 | 20.5 |
| Reference | 6.1 | 20.0 |
| 28 | 6.2 | 20.5 |
| 28 | 9.4 | 26.5 |
| 29 | 8.3 | 18.8 |
| 29 | 7.9 | 17.0 |
| 30 | 13.5 | 41.0 |
| 30 | 14.0 | 36.0 |

**Table 10: Results from a two-way ANOVA on the texture attributes Brookfield and Shear stress for strain B and strain D under varying cfu's.**

| | Brookfield viscosity | | Shear stress | |
|---|---|---|---|---|
| Term | Coefficient | *P*-value | Coefficient | *P*-value |
| Mean reference (intercept) | 6059 | | 20 | |
| Strain B | 1733 | 0.0244 | 3 | 0.0277 |
| Strain D | 2057 | 0.0318 | -2 | 0.2596 |
| Interaction between strain B+D | 23192 | 0.0048 | 71 | 0.0019 |

In both the Brookfield measurements as well as the shear stress measurements it was found that the addition of strain B had a significant positive effect (i.e. the coefficient - *P* < 0.05) and that strain B and strain D have a significant positive interaction effect (i.e. the coefficient - *P* < 0.05). Strain D has a significant positive effect on the texture attribute Brookfield but not on the shear stress.

This analysis demonstrates that strain B and strain D have synergistic effects on the texture attributes Brookfield and shear stress that cannot be explained by simple additive effects alone. In case of a simple additive effect. The coefficient for the interaction between strain B+D would be zero.

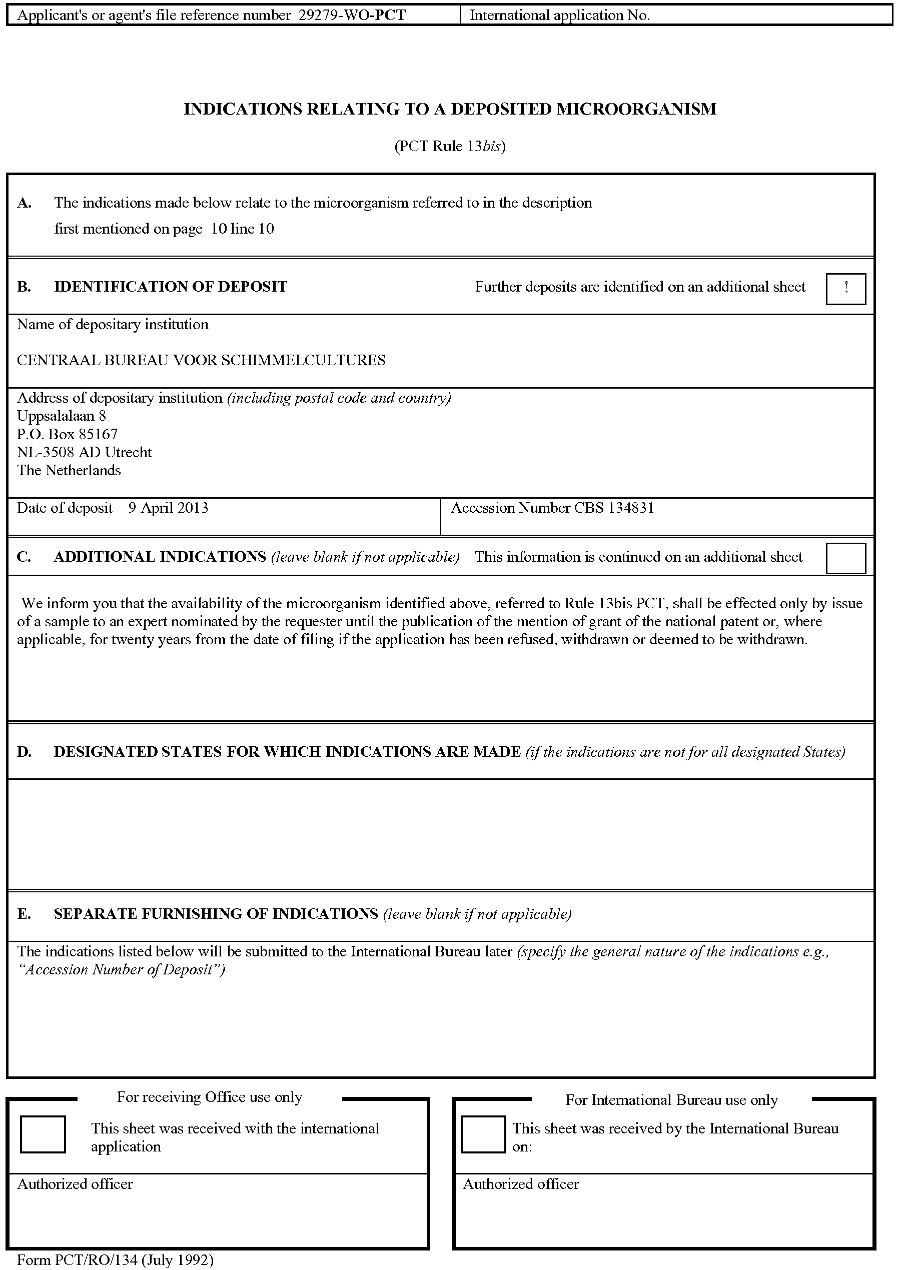

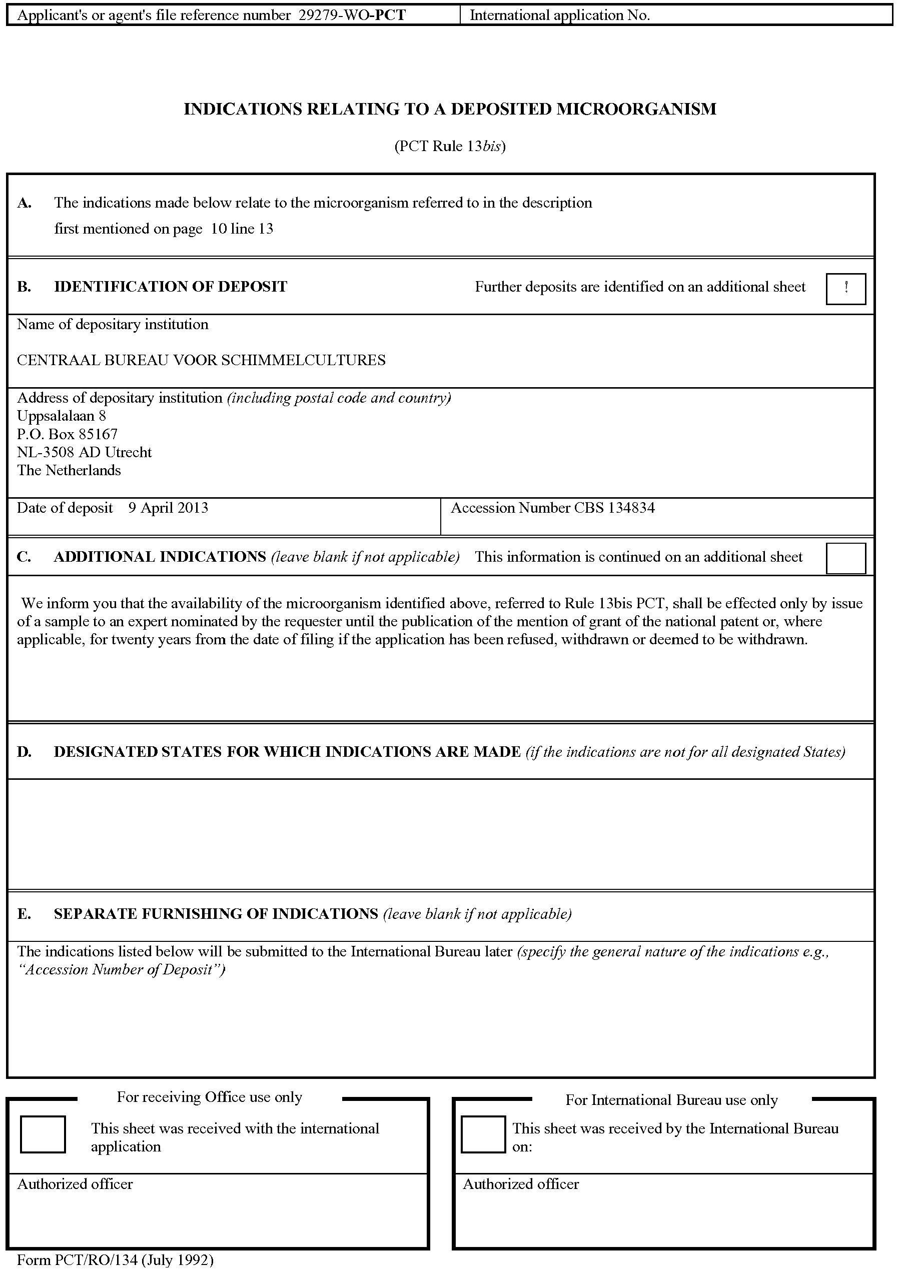

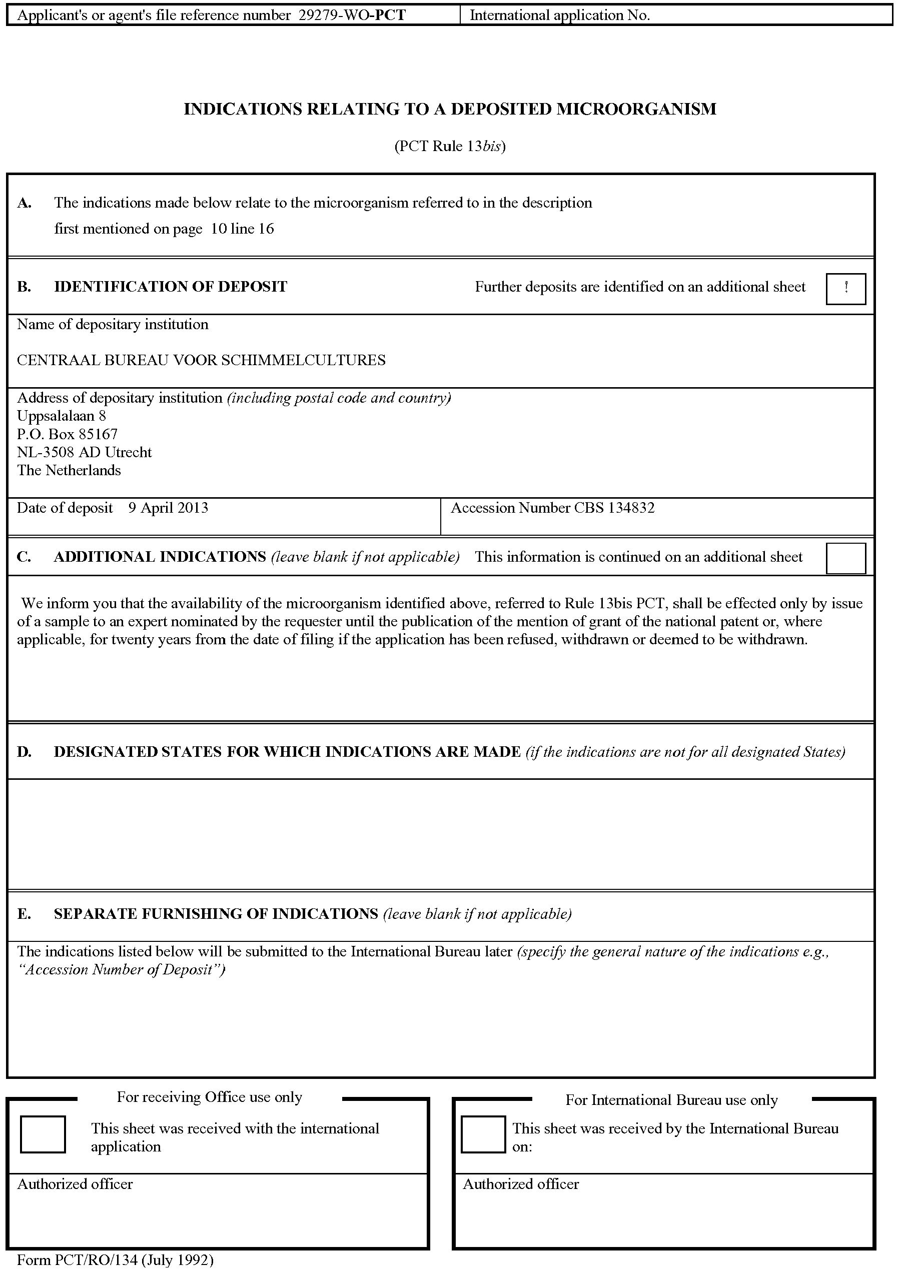

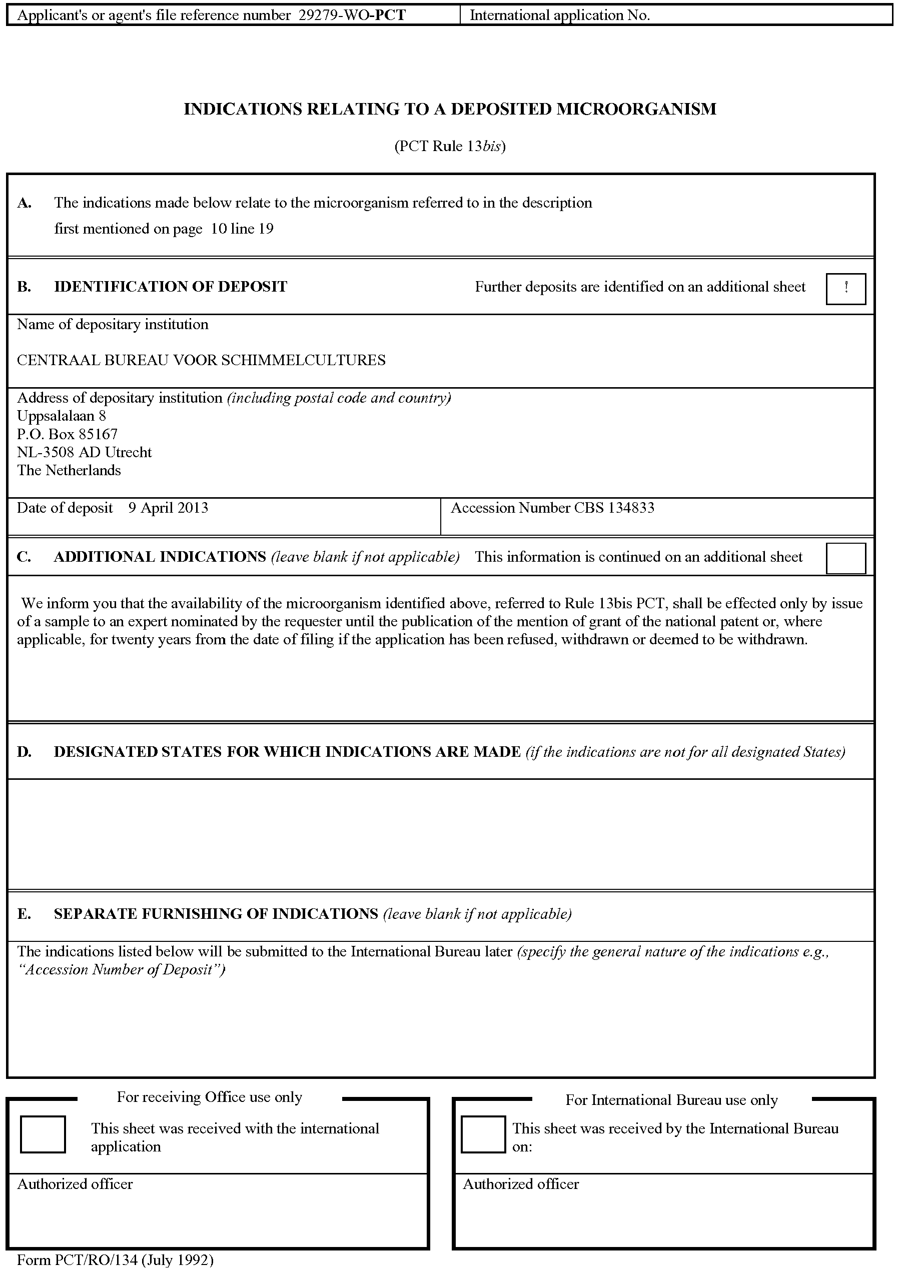

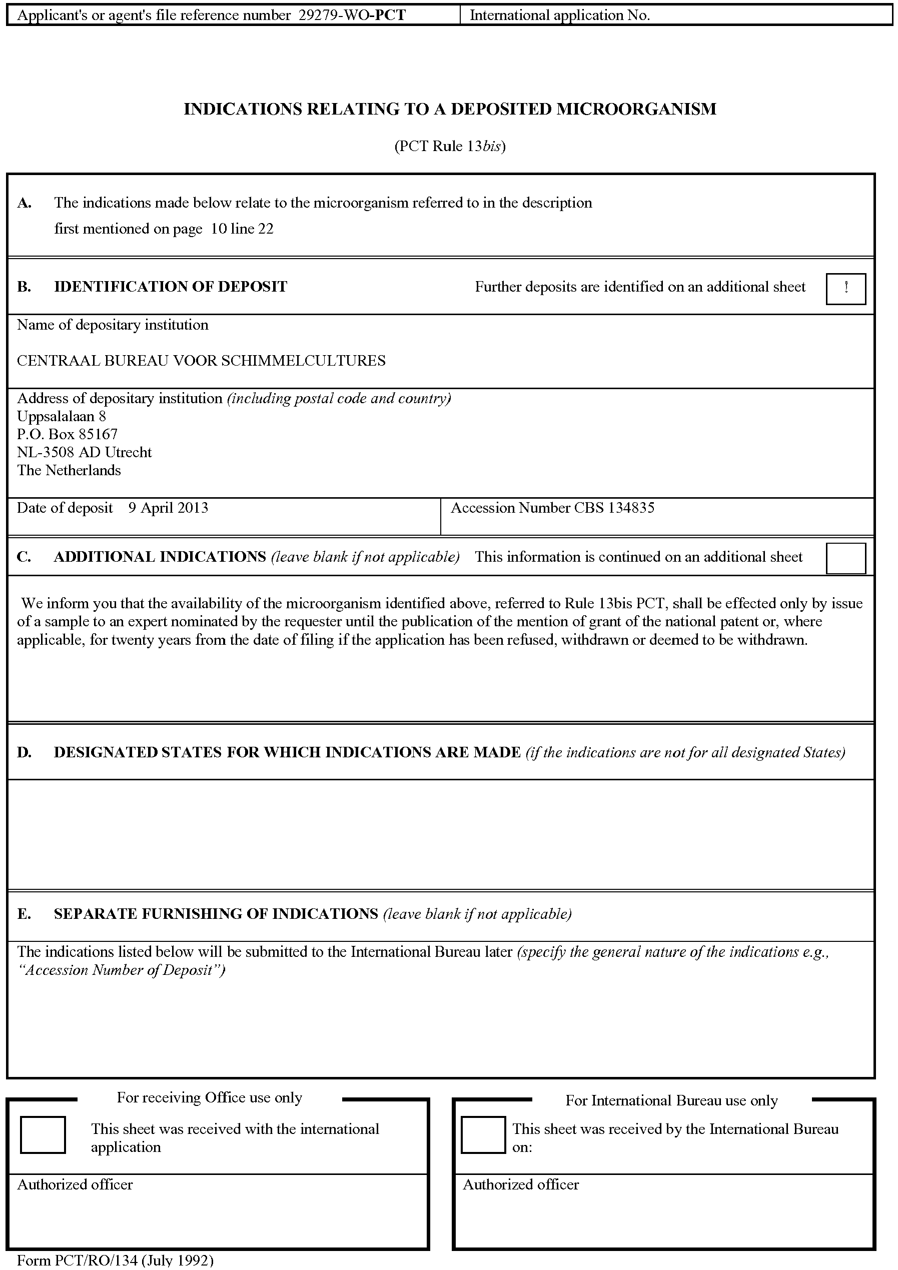

## Claims

1. A composition comprising *Streptococcus thermophilus* DS71586 (strain B) and *Streptococcus thermophilus* DS71585 (strain D).

2. A composition according to claim 1, wherein the composition further comprises a *Streptococcus thermophilus* DS71579 (strain A) and / or *Streptococcus thermophilus* DS71584 (strain C).

3. A composition according to claim 1 or 2, further comprising a *Lactobacillus delbrueckii ssp. bulgaricus* strain.

4. A composition according to claim 3, wherein the *Lactobacillus delbrueckii ssp. Bulgaricus* strain is *Lactobacillus delbrueckii ssp. bulgaricus* DS71836 (strain E).

5. A composition according to claim 4, comprising *Streptococcus thermophilus* DS71579 (strain A) and *Streptococcus thermophilus* DS71586 (strain B) and *Streptococcus thermophilus* DS71584 (strain C) and *Streptococcus thermophilus* DS71585 (strain D) and *Lactobacillus delbrueckii ssp. bulgaricus* DS71836 (strain E).

6. A composition according to claim 5, wherein the *Streptococcus thermophilus* strain each are present in an amount of 24,5% based on the total cfu's and *Lactobacillus delbrueckii ssp. bulgaricus* DS71836 is present in an amount of 1% based on the total cfu's.

7. *Streptococcus thermophilus* selected from the group consisting of DS71579 deposited with the Centraalbureau voor Schimmelcultures on 9 April 2013 having deposition number CBS134831, DS71586 deposited with the Centraalbureau voor Schimmelcultures on 9 April 2013 having deposition number CBS134834, DS71584 deposited with the Centraalbureau voor Schimmelcultures on 9 April 2013 having deposition number CBS134832 and DS71585 deposited with the Centraalbureau voor Schimmelcultures on 9 April 2013 having deposition number CBS134833.

8. *Lactobacillus delbrueckii ssp. bulgaricus* DS71836 deposited with the Centraalbureau voor Schimmelcultures on 9 April 2013 having deposition number CBS134835.

9. A process for the production of a fermented milk product comprising fermenting milk using any of the compositions as defined by anyone of claims 1-6 and wherein the texture of the fermented milk product obtained has been improved compared to the texture of a fermented milk product that has not been produced using any of the composition as defined by anyone of claims 1-6.

10. A process according to claim 9, wherein the rheology of the fermented milk product has been improved.

11. A process according to claim 8 or claim 9, wherein the fermented milk product is yogurt.

12. A process according to any of the claims 9 to 11, wherein the Brookfield and/or the shear stress of the fermented milk product has been improved.

13. A fermented milk product obtainable by the process of claims 9-12 and comprising a composition as defined in any of claims 1-6 **characterized in that** the fermented milk product has an improved texture as defined in claim 9 or 10 compared to a fermented milk product that has not been produced by the process of claim 9 or 10 and is not comprising a composition as defined in any of claims 1-6.

14. A fermented milk product according to claim 13, wherein the fermented milk product is yogurt.

15. Use of any of the compositions as defined by claims 1-6 for the production of the fermented milk product.

## Patentansprüche

1. Zusammensetzung, umfassend *Streptococcus thermophilus* DS71586 (Stamm B) und *Streptococcus thermophilus* DS71585 (Stamm D).

2. Zusammensetzung nach Anspruch 1, wobei die Zusammensetzung ferner *Streptococcus thermophilus* DS71579 (Stamm A) und/oder *Streptococcus thermophilus* DS71584 (Stamm C) umfasst.

3. Zusammensetzung nach Anspruch 1 oder 2, ferner umfassend einen *Lactobacillus delbrueckii ssp. bulgaricus-Stamm.*

4. Zusammensetzung nach Anspruch 3, wobei es sich bei dem *Lactobacillus delbrueckii ssp. bulgaricus-Stamm* um *Lactobacillus delbrueckii ssp. bulgaricus* DS71836 (Stamm E) handelt.

5. Zusammensetzung nach Anspruch 4, umfassend *Streptococcus thermophilus* DS71579 (Stamm A) und *Streptococcus thermophilus* DS71586 (Stamm B) und *Streptococcus thermophilus* DS71584 (Stamm C) und *Streptococcus thermophilus* DS71585 (Stamm D) und *Lactobacillus delbrueckii ssp. bulgaricus* DS71836 (Stamm E).

6. Zusammensetzung nach Anspruch 5, wobei die *Streptococcus thermophilus-Stämme* jeweils in einer Menge von 24,5% bezogen auf die cfu-Gesamtzahl vorliegen und *Lactobacillus delbrueckii ssp. bulgaricus* DS71836 in einer Menge von 1% bezogen auf die cfu-Gesamtzahl vorliegt.

7. *Streptococcus thermophilus,* ausgewählt aus der Gruppe bestehend aus DS71579, hinterlegt beim Centraalbureau voor Schimmelcultures am 9. April 2013 mit Hinterlegungsnummer CBS134831, DS71586, hinterlegt beim Centraalbureau voor Schimmelcultures am 9. April 2013 mit Hinterlegungsnummer CBS134834, DS71584, hinterlegt beim Centraalbureau voor Schimmelcultures am 9. April 2013 mit Hinterlegungsnummer CBS134832, und DS71585, hinterlegt beim Centraalbureau voor Schimmelcultures am 9. April 2013 mit Hinterlegungsnummer CBS134833.

8. *Lactobacillus delbrueckii ssp. bulgaricus* DS71836, hinterlegt beim Centraalbureau voor Schimmelcultures am 9. April 2013 mit Hinterlegungsnummer CBS134835.

9. Verfahren zur Erzeugung eines vergorenen Milchprodukts, umfassend Vergären von Milch unter Verwendung einer der Zusammensetzungen mit der in einem der Ansprüche 1-6 angegebenen Bedeutung, und wobei die Konsistenz des erhaltenen vergorenen Milchprodukts verglichen mit der Konsistenz eines vergorenen Milchprodukts, das nicht unter Verwendung einer der Zusammensetzungen mit der in einem der Ansprüche 1-6 angegebenen Bedeutung erzeugt wurde, verbessert wurde.

10. Verfahren nach Anspruch 9, wobei die Rheologie des vergorenen Milchprodukts verbessert wurde.

11. Verfahren nach Anspruch 8 oder Anspruch 9, wobei es sich bei dem vergorenen Milchprodukt um Joghurt handelt.

12. Verfahren nach einem der Ansprüche 9 bis 11, wobei die Brookfield- und/oder die Scherspannung des vergorenen Milchprodukts verbessert wurde.

13. Vergorenes Milchprodukt, erhältlich mit dem Verfahren gemäß Anspruch 9-12 und umfassend eine Zusammensetzung mit der in einem der Ansprüche 1-6 angegebenen Bedeutung, **dadurch gekennzeichnet, dass** das vergorene Milchprodukt eine verbesserte Konsistenz gemäß Anspruch 9 oder 10 verglichen mit einem vergorenen Milchprodukt, das nicht mit dem Verfahren gemäß Anspruch 9 oder 10 erzeugt wurde und keine Zusammensetzung mit der in einem der Ansprüche 1-6 angegebenen Bedeutung umfasst, aufweist.

14. Vergorenes Milchprodukt nach Anspruch 13, wobei es sich bei dem vergorenen Milchprodukt um Joghurt handelt.

15. Verwendung einer der Zusammensetzungen mit der in Anspruch 1-6 angegebenen Bedeutung zur Erzeugung des vergorenen Milchprodukts.

## Revendications

1. Composition comprenant *Streptococcus thermophilus* DS71586 (souche B) et *Streptococcus thermophilus* DS71585 (souche D).

2. Composition selon la revendication 1, où la composition comprend en outre *Streptococcus thermophilus* DS71579 (souche A) et/ou *Streptococcus thermophilus* DS71584 (souche C).

3. Composition selon la revendication 1 ou 2, comprenant en outre une souche de *Lactobacillus delbrueckii* ssp. *bulgaricus.*

4. Composition selon la revendication 3, où la souche de *Lactobacillus delbrueckii* ssp. *bulgaricus* est *Lactobacillus delbrueckii* ssp. *bulgaricus* DS71836 (souche E).

5. Composition selon la revendication 4, comprenant *Streptococcus thermophilus* DS71579 (souche A), et *Streptococcus thermophilus* DS71586 (souche B), et *Streptococcus thermophilus* DS71584 (souche C), et *Streptococcus thermophilus* DS71585 (souche D), et *Lactobacillus delbrueckii* ssp. *bulgaricus* DS71836 (souche E).

6. Composition selon la revendication 5, où les souches de *Streptococcus thermophilus* sont présentes chacune selon une quantité de 24,5% sur la base des ufc totales et *Lactobacillus delbrueckii* ssp. *bulgaricus* DS71836 est présente selon une quantité de 1% sur la base des ufc totales.

7. *Streptococcus thermophilus* choisi dans le groupe constitué par DS71579 déposé auprès du Centraalbureau voor Schimmelcultures le 9 avril 2013 sous le numéro de dépôt CBS134831, DS71586 déposé auprès du Centraalbureau voor Schimmelcultures le 9 avril 2013 sous le numéro de dépôt CBS134834, DS71584 déposé auprès du Centraalbureau voor Schimmelcultures le 9 avril 2013 sous le numéro de dépôt CBS134832, et DS71585 déposé auprès du Centraalbureau voor Schimmelcultures le 9 avril 2013 sous le numéro de dépôt CBS134833.

8. *Lactobacillus delbrueckii* ssp. *bulgaricus* DS71836 déposé auprès du Centraalbureau voor Schimmelcultures le 9 avril 2013 sous le numéro de dépôt CBS134835.

9. Procédé de production d'un produit de lait fermenté, comprenant la fermentation de lait en utilisant l'une quelconque parmi les compositions telles que définies selon l'une quelconque des revendications 1-6, et où la texture du produit de lait fermenté obtenu a été améliorée par rapport à la texture d'un produit de lait fermenté n'ayant pas été produit en utilisant l'une quelconque parmi les compositions telles que définies selon l'une quelconque des revendications 1-6.

10. Procédé selon la revendication 9, où la rhéologie du produit de lait fermenté a été améliorée.

11. Procédé selon la revendication 8 ou la revendication 9, où le produit de lait fermenté est un yaourt.

12. Procédé selon l'une quelconque des revendications 9 à 11, où la valeur Brookfield et/ou la contrainte de cisaillement du produit de lait fermenté a été améliorée.

13. Produit de lait fermenté pouvant être obtenu par le procédé selon les revendications 9-12 et comprenant une composition telle que définie selon l'une quelconque des revendications 1-6, **caractérisé en ce que** le produit de lait fermenté possède une texture améliorée telle que définie selon la revendication 9 ou 10 par rapport à un produit de lait fermenté n'ayant pas été produit par le procédé selon la revendication 9 ou 10 et ne comprenant pas de composition telle que définie selon l'une quelconque des revendications 1-6.

14. Produit de lait fermenté selon la revendication 13, où le produit de lait fermenté est un yaourt.

15. Utilisation de l'une quelconque parmi les compositions telles que définies selon les revendications 1-6, pour la production du produit de lait fermenté.
